Europäisches Patentamt

(19) European Patent Office

Office européen des brevets

(11) Publication number: **0 169 068**
A2

# (12) EUROPEAN PATENT APPLICATION

(21) Application number: 85305109.2

(22) Date of filing: 17.07.85

(51) Int. Cl.⁴: **C 12 F 3/06**, C 12 P 7/06,
C 12 C 5/00

(30) Priority: 20.07.84 GB 8418506

(43) Date of publication of application: 22.01.86
Bulletin 86/4

(84) Designated Contracting States: BE CH DE FR GB LI NL

(71) Applicant: THE BREWING RESEARCH FOUNDATION,
Lyttel Hall Nutfield, Redhill Surrey RH1 4HY (GB)

(72) Inventor: Laws, Derek Roy James, Dr., 2A First Avenue,
Bexley Heath Kent (GB)
Inventor: Waites, Michael John, Dr., 28 The Covey, Pound
Hill Crawley Sussex (GB)

(74) Representative: Brock, Peter William et al, Michael
Burnside & Partners 2 Serjeants' Inn Fleet Street,
London EC4Y 1HL (GB)

(54) Utilization of spent grains.

(57) Spent grains and waste products from malting process can be converted into valuable fermentable materials by hydrolysis at elevated temperature and at atmospheric or, more preferably, superatmospheric pressure. Acids (preferably sulphuric acid) and bases can be used as hydrolyzing agent, and a fermentable product is formed by hydrolysis of the starch, 3-glucans, pentosans and other hemicellulosic material in the spent grains.

The fermentable product can be added to wort or to a masking liquid for fermentation, or can be fermented by itself.

## UTILIZATION OF SPENT GRAINS

This invention concerns the utilization of spent grains from the brewing and distilling industries and waste products from malting processes. More particularly, this invention concerns the conversion of spent grains and waste products from malting processes into a fermentable product, and in one embodiment, the use of the fermentable product in a fermentation process, for instance a process for producing an alcoholic beverage.

Malted barley is the major source of carbohydrates used by the brewing industry. After the usual extraction of fermentable carbohydrates by mashing malt with warm water and separating the resulting wort, the residue comprises spent grains, which generally contain about 80 weight % of moisture. These spent grains are usually collected from British breweries by specialist companies who sell them, without further processing, to farmers as low grade cattle food. Breweries, particularly those sited in large towns, are finding it increasingly difficult to dispose of spent grains, and most breweries experience problems during the Summer and Autumn, when plenty of other cattle food is available. Spent grains are costly to dry, and the added value of the dried product does not pay for the cost of fuel. Moulds and other microorganisms rapidly grow on wet spent grains, leading to a reduction in value, increased difficulties in handling, and unpleasant smells. A typical composition of dried brewery spent grains is shown in Table 1. The distilling industry is also a substantial producer of spent grains which are generally similar to those produced by the brewing industry and may be treated in the same way according to the present invention.

TABLE 1

Composition of Dried Spent Grains (wt%)

| | |
|---|---|
| Moisture | 2.3 |
| Lipids | 10.6 |
| Protein | 21.2 |
| Cellulose | 10.9 |
| Lignin | 6.5 |
| $\beta$-Glucans, starch, pentosans etc. | 43.7 |
| Free sugars | 1.4 |
| Ash | 3.4 |
| | 100.0 |
| | ======= |

Ways of using spent grains and their derivatives in baking and as meat extenders have been examined. A product, said to be useful as a foam stabilizer can be produced by the hydrolysis of spent grains, employing acid or alkaline conditions (US-A-2 806 791) or water at superatmospheric pressure and temperatures above 100°C (US-A-3099563). The foam stabilization effect is possibly due to hydrolyzed proteins or to melanoidins formed by reaction of the hydrolyzed proteins with lower carbohydrates, especially in the presence of acids or alkalis (see US-A-2 806 791, col. 2 lines 18 to 29). Small quantities of the product (0.005 to 0.1% by weight) are added to beer, after fermentation, as a foam stabiliser (US-A-2 806 791, col. 4, lines 22 to 26).

The present invention provides a process for the improved utilisation of spent grains and/or of waste products from the malting process (e.g. barley and malt dust, rootlets and screenings), which are converted into a fermentable broth leaving a solid residue containing increased proportions of proteins and lipids.

The present invention provides a method for the utilization of residual carbohydrates in spent grains and/or malting waste products, which comprises subjecting said spent grains and/or malting waste products to hydrolysis at elevated temperature, and collecting a fermentable product.

According to a preferred embodiment of the invention, hydrolysis is carried out under acidic or basic conditions at superatmospheric pressure, at a temperature of 140 to 180°C e.g. in the presence of 1 to 2 wt.% of sulphuric acid.

According to another embodiment of the invention, hydrolysis is carried out under acidic or basic conditions at atmospheric pressure, e.g. by boiling with from 1 to 30 wt.% of sulphuric acid for 1 to 6 hours, the product preferably being filtered to remove insoluble materials.

The fermentable product can be subjected to an alcoholic fermentation. The fermentable product can, for instance, be added to a wort for fermentation, or can be added to or used as a mashing liquor, or can be fermented by itself.

In the accompanying Drawings:

Figure 1 is a graph illustrating the manner in which the amount of fermentable glucose in the hydrolysis product varies with the hydrolysis temperature; and

Figure 2 is a diagrammatic illustration of one way of carrying out the process of the invention.

The process of the invention involves hydrolysis of the spent grains or malting waste products, separation of the hydrolyzate from the solid residue, and, in some instances, contacting the hydrolyzate with an absorbent, such as active charcoal, to remove coloured contaminants such as the melanoidins referred to in US-A-2 806 791, and leave a pale-coloured hydrolyzate containing fermentable carbohydrates.

In one preferred embodiment of the invention, the hydrolyzate is employed to supplement wort. It can, for example, be added to the kettle, the hop separator, whirlpool separator or fermentation vessel. In this way, a considerable proportion, e.g. 5 to 15% of the fermentable carbohydrate employed in a brewing process can be replaced by the hydrolyzate, and surprisingly it has been found that beer produced in this way is not detectably different in flavour and other properties from a comparison brew made without addition of hydrolyzate. In another embodiment, the hydrolyzate can be recycled and used in the mashing process. In yet another embodiment, the hydrolyzate can itself be fermented to give an alcohol solution, from which ethanol can be recovered, e.g.by distillation.

The hydrolyzate obtained according to the present invention (alternatively referred to as the fermentable broth) is obtained by converting the starch, $\beta$-glucans, pentosans and other hemicellulosic material present in spent grains into a mixture of fermentable carbohydrates by acid hydrolysis, alkaline hydrolysis or enzymic treatment or by a combination of these treatments (see Examples 1 to 6). The broth obtained by acid hydrolysis contains nitrogenous compounds in addition to fermentable carbohydrates. (See Table 2 for a typical composition).

## TABLE 2

Analysis of a Broth obtained by Acid Hydrolysis of
Spent Grains with 10% sulphuric acid
at Atmospheric Pressure
(g/100g Spent Grain on a Dry Weight Basis)

| | |
|---|---|
| Total Carbohydrates | 37 |
| Arabinose | 4 |
| Xylose | 7 |
| Glucose | 24 |
| Lipids | $0.4 \times 10^{-3}$ |
| Total Nitrogen | 1.11 |
| $\alpha$-Amino Nitrogen | 0.46 |
| Ash | 1.1 |
| Colour (EBC units)* | $>$ 150 |

*

European Brewery Convention Units (see "Analytica-E.B.C."
3rd Edition, Sections E29 to E31, European Brewery
Convention, Published by Schweizer Brauerei-Rundschau,
Zurich, 1975).

The broth provides a raw material for the fermentation industries and after any necessary decolorization, it is particularly suitable for use in brewing. Alternatively the broth can be used as a total or partial replacement of the treated water (mashing liquor) used for mashing a grist consisting of barley malt, often together with cereal adjuncts such as wheat flour, maize, or raw barley. Alternatively, it can itself be fermented to produce a solution for ethanol recovery.

During hydrolysis it is possible to solubilize up to 65% of the spent grains (dry weight basis). The residue remaining is rich in lipids, cellulose, proteins and lignin.

A typical composition of a dried residue, obtained by the acid hydrolysis of spent grain is shown in Table 3.

## TABLE 3

Composition of a Dried Residue from Spent Grains (%)
(g/100g of spent grains hydrolyzed on a dry weight basis)

| | | |
|---|---|---|
| Moisture | 0.7 | (1.5)* |
| Lipid | 8.5 | (19.1) |
| Protein | 12.2 | (27.6) |
| Cellulose | 12.7 | (28.7) |
| Lignin | 7.0 | (15.8) |
| Free Sugars | 0.2 | (0.5) |
| Ash | 3.0 | (6.7) |
| | 44.3 | (100.0) |

*Figures in parenthesis are the percentage composition

Such residues contain higher levels of proteins and lipids than do spent grains, and are of interest to manufacturers of animal feed. Alternatively, lipids could be removed from the residues by solvent extraction, or by treatment with liquid carbon dioxide (see Example 7), to provide edible oils or a raw material for the manufacture of soap. The material remaining after extraction of lipids would still be of value for use in animal feeds.

The preferred method of carrying out the invention involves acid hydrolysis of spent grains either at atmospheric or, more preferably, at superatmospheric pressure. Mineral acids such as hydrochloric, phosphoric or sulphuric acid can be used. Sulphuric acid is preferred, however, because at the end of hydrolysis, calcium hydroxide can be added, the resulting insoluble sulphates being removed from the broth by filtration. If hydrochloric acid is employed, the broth contains high levels of soluble chlorides, which can be removed by treatment with suitable ion-exchange resins.

Concentrations of 1 to 30% of sulphuric acid can be used to effect the hydrolysis by boiling at atmospheric pressure. At acid concentrations above 30% charring occurs and any fermentable sugars are rapidly degraded.

The period of boiling the reaction mixture can be from 1 to 6 hours, depending on the concentration of sulphuric acid. The preferred reaction conditions at atmospheric pressure involve boiling spent grains for about 2 hours with 5 to 10% sulphuric acid, under these conditions, the formation of fermentable carbohydrates can be readily controlled. At the end of heating, an aqueous slurry of calcium hydroxide is added to the hot reaction mixture, which is filtered without cooling, the resulting calcium sulphate acting as filter aid. The resulting brown coloured hydrolysate (broth) is a suitable raw material for many fermentation industries (see Table 2). If the broth is going to be used in brewing, however, it is subsequently filtered through a bed of charcoal to remove coloured materials, some nitrogenous components, and volatile compounds such as furfural, to give a pale amber-coloured liquid containing fermentable carbohydrates. A typical composition of a broth prepared in this way is given in Example 1. Experiments have shown that over 20g of glucose can be obtained by acid hydrolysis of 100g of spent grains (on a dry weight basis). The non-fermentable sugars arabinose and xylose are also formed during acid hydrolysis. The yield of total fermentable sugars obtained by acid hydrolysis of spent grains could be substantially increased if arabinose and xylose could be utilised by yeast. Xylose could be converted into the fermentable sugar xylulose by treating the broth with glucose isomerose.

There is an advantage in effecting the hydrolysis of spent grains at elevated temperature in a pressure vessel, a consequent reduction in the quantity of calcium hydroxide added at the end of the reaction. Thus, if the reaction is carried out at 150°C, a concentration of 1 to 2% of sulphuric acid is sufficient to produce a similar yield of fermentable carbohydrates to that obtained at atmospheric pressure using 5 to 10% of acid (see Example 3). In addition, it is possible to reduce by over 50% the reaction time when hydrolyzing spent grains at elevated temperature, e.g. to from 15 to 20 minutes, without any adverse effect on the yield of fermentable carbohydrates. A further advantage of carrying out the hydrolysis at superatmospheric pressure is that the product is sufficiently pale in colour not to need a special decolorizing treatment. A typical composition of a broth prepared in this way is shown in Example 3.

It is found that when hydrolysis is carried out at superatmospheric pressure, a good yield of the fermentable monosaccharide, glucose is obtained at 120 to 200°C, more preferably 140 to 180°C, and most preferably at 150 to 170°C.

Hydrolysis of spent grain can also be carried out under alkaline conditions with the subsequent addition of enzymes e.g. cellulase. The complex of cellulase enzymes usually contains a mixture of $\beta$-glucanases and $\beta$-glucosidases. Under these conditions, a proportion of the cellulose is degraded to glucose, and some degradation of gums, pentosans and other hemicelluloses will also occur. The multistage process involves boiling spent grains with aqueous alkali, preferably sodium or potassium hydroxide, for about 3 hours, followed by addition of hydrochloric acid, a fivefold dilution with water, and finally digestion with cellulase over 16 to 20 hours (see Example 6). After removing the solids by filtration or centrifugation, a dilute dark brown broth is obtained. A broth obtained in this way was concentrated by evaporation and its composition is shown in Example 6. The overall yield of fermentable carbohydrates is generally similar to that obtained by acid hydrolysis of spent grains, but more of the proteins present in spent

- 9 -                    0169068

grains are solubilized during alkaline hydrolysis.

Broths produced by acid or alkaline hydrolysis of spent grains should provide suitable feedstock for the biotechnology industry, e.g. for the production of alcohol, food or fodder yeasts and for the production of pharmaceuticals and other products involving growth of microorganisms. In particular, the broth obtained by acid hydrolysis of spent grains, can be used with advantage for the production of beer. Thus hydrolysis of all the spent grains from a brew provides a broth containing sufficient fermentable carbohydrates and nitrogenous components to replace 5 to 15% of the malt required for a subsequent similar brew. Alternatively this broth can be used to extend the capacity of brewery mashing equipment. Beers produced from worts containing a proportion of the spent grain broth were of similar quality and flavour to those of control beers produced from an all-malt grist. (See Example 8).

Alternatively, the broth can itself be fermented, producing a solution of ethanol, from which the ethanol can be separated by conventional means, such as distillation. Example 9 illustrates this possibility.

### Examples

The following Examples (in which percentages are by weight unless otherwise indicated) illustrate various aspects of the invention but are not intended to limit the scope of this invention.

## EXAMPLE 1

### Acid Hydrolysis of Spent Grain at Atmospheric Pressure

Wet spent grains (11.8 kg at ca 83% moisture) from a pilot brewery mash tun were placed in a stainless steel vessel (30 cm deep by 38 cm diameter). A solution of sulphuric acid (570 ml of sulphuric acid of specific gravity 1.84 plus 7 litres of water) was added to the spent grains (final acid concentration of 5% w/w). The slurry was brought to the boil whilst stirring and then simmered for a period of 1.5 hours with occasional stirring. Calcium hydroxide (800 g in 1 litre of water) was then added to the hot acidic slurry, which was then filtered using a Buchner funnel (Whatman No. 3 filter paper). The resulting filtrate (11 litres), which was a dark green/brownish liquid (pH 4.75) was filtered through activated charcoal (300g) giving a spent grain broth that was pale amber in colour. This broth was stored overnight at 4°C and adjusted to pH 5 with the addition of 2M sodium hydroxide (60 ml). The composition of the broth is shown in Table 4.

## TABLE 4

Analysis of a Decolorized Broth Obtained by Acid
Hydrolysis Using 5% Sulphuric Acid
(g/100g Spent Grains on a Dry Weight Basis)

| | |
|---|---|
| Total Carbohydrate | 22.5 |
| Arabinose | 4.3 |
| Xylose | 5.5 |
| Glucose | 8.5 |
| Lipid Content | $0.4 \times 10^{-3}$ |
| Total Nitrogen | 0.33 |
| $\alpha$-Amino Nitrogen | 0.11 |
| Colour (EBC units) | 0.5 |

Hydrolysis carried out in a glass vessel employing more
vigorous stirring produced 14 g of glucose per 100 g of
spent grains.

## EXAMPLE 2

### Acid Hydrolysis of Malting Waste Materials at
### Atmospheric Pressure

Samples (100 g) of malt dust or rootlets at 3-5%
moisture were mixed with 600 ml of 10%, w/w sulphuric acid
and boiled under reflux for 2 hours with continuous
stirring. The hydrolysate was adjusted to pH 5.0 with
calcium hydroxide, and the mixture was filtered through
Whatman No. 3 filter paper using a Buchner funnel. The
residue was washed with deionised water until the final
volume of filtrate was 1.5 litres. The composition of the
resulting brown coloured broth is given in Table 5.

TABLE 5

Analysis of Broths obtained by Acid Hydrolysis of
Dust and Rootlets at Atmospheric Pressure
(g/100g Dust or Rootlets on a Dry Weight Basis)

|  | Dust | Rootlets |
|---|---|---|
| Total Carbohydrates | 28.5 | 28.5 |
| Arabinose | 3.5 | 3.3 |
| Xylose | 7.6 | 4.5 |
| Glucose | 9.0 | 15.4 |
| Total Nitrogen | 3.4 | 3.7 |
| $\alpha$-Amino Nitrogen | 0.9 | 1.0 |
| Colour (EBC units) | 36.1 | 37.8 |

## EXAMPLE 3
Acid Hydrolysis of Spent Grains at Elevated Temperature

Hydrolysis of spent grains at 150°C was carried out using a Baskerville and Lindsay autoclave fitted with a reaction vessel of 1.0 litre capacity. A sample of wet spent grain (500g) was placed in the vessel and a solution of sulphuric acid (5.4 ml, specific gravity 1.84; plus 495 ml of water) was added to give a final acid concentration of 1% w/w. The mixture was heated from 20 to 150°C over a period of one and a quarter hours and then held at 150 to 160°C for half an hour. The vessel was cooled using a water cooling coil over a period of one and a half hours until the temperature had dropped to 100°C, when the hydrolysis was decanted and further cooled to ambient temperature. The reaction mixture was filtered using a Buchner funnel (Whatman No. 3 filter paper) and the pH of the filtrate (broth) was adjusted to 5.15 by the addition of 4M sodium hydroxide (35 ml). The final volume of the broth was 1150 ml and its composition is shown in Table 6.

## TABLE 6

Composition of a Broth Obtained by the Hydrolysis of
Spent Grains at 150°C (g/100g Spent Grains on a
Dry Weight Basis)

| | |
|---|---|
| Total Carbohydrates | 48.3 |
| Arabinose | 5.2 |
| Xylose | 6.4 |
| Glucose | 13.4 |
| Total Nitrogen | 1.6 |
| α-Amino Nitrogen | 0.3 |

## EXAMPLE 4

Acid Hydrolysis of Spent Grains at Elevated
Temperatures (Batch System)

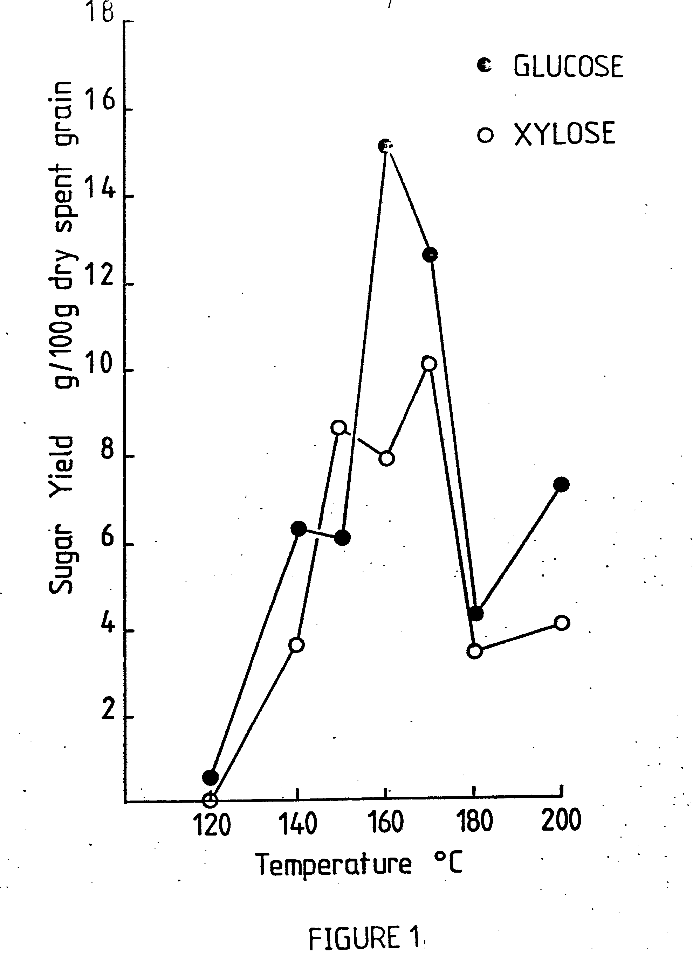

A stainless steel reaction vessel (length 23.0 cm, diameter 4.5 cm, volume 160 ml) which was fitted with a cap containing a pressure relief valve was used for these studies. A portion of spent grains (75 g) was placed in the vessel together with water (74.2 ml) and concentrated sulphuric acid (0.8 ml, specific gravity 1.84) to give a final acid concentration of 1% w/w. The reaction vessel was immersed for 20 minutes in an oil bath heated to 160°C. The vessel was then removed from the bath, cooled by immersion in water (15°C), and opened, and the contents were poured into a beaker. The pH of the mixture was adjusted to 5.0 by addition of solid calcium hydroxide and the mixture was filtered (Whatman number 3 filter paper). The filter cake was washed with water (50 ml to 100 ml) to give a filtrate of total volume 200 to 250 ml. Hyrolyses were carried out in a similar way over the temperature range from 120° to 200°C. The levels of glucose and xylose present in the filtrates are shown in Figure 1 of the accompanying Drawings. The maximum level of the desired glucose was obtained by hydrolysis of the spent grains at approximately 160°C.

## EXAMPLE 5

### Acid Hydrolysis of Spent Grains at Elevated Temperature (Continuous System)

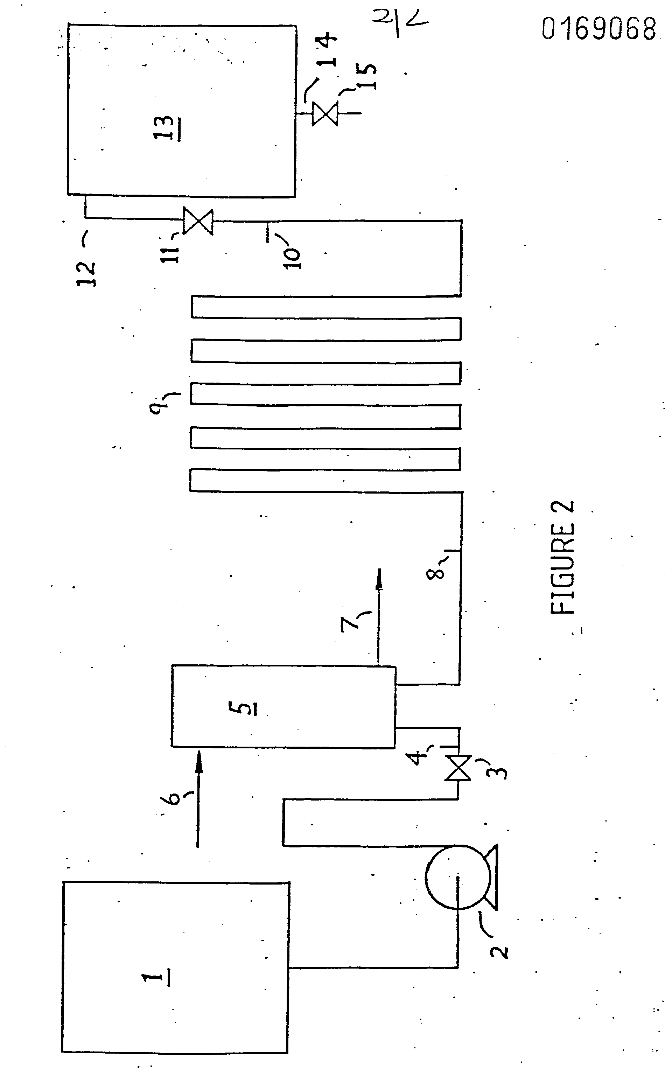

The apparatus used in this Example is shown diagrammatically in Figure 2 of the accompanying Drawings. This apparatus comprises a storage tank 1 of 60 litres capacity provided with a stirrer (not shown) and appropriate means (also not shown) for introducing solids and liquids. It is connected to a calandria or other suitable pressure vessel 5 by way of a line provided with a centrifugal pump 2, a first valve 3, and a first pressure gauge 4. Steam from line 6 passes through the calandria 5 leaving by line 7. After passing through the calandria 5, slurries or liquids are transferred past thermometer 8, through holding coils 9 and thence to receiving vessel 13 by way of line 12 having a second valve 11 and a second pressure gauge 10. The contents of receiving vessel 13 leave it through outlet 14 provided with a third valve 15.

Wet spent grains (24 kg at about 80% moisture) were collected after mashing and immediately milled using a disc mill (Condux-Werk). The storage vessel 1 was filled with water which was then heated to 70°C. The first valve 3 was shut, the centrifugal pump 2 was switched on and steam (at 0.65 MPa, 160°C) was admitted to the Calandria 5 through line 6 leaving by line 7. Water from the storage vessel 1 was allowed to flow through the system by opening the first and second valves 3 and 11 and permitted to run to waste from the receiving vessel 13 with third valve 15 open. This procedure primed and preheated the apparatus and was continued until 13 litres of water remained in the storage vessel 1 when the first and second valves 3 and 11 were closed and the pump 2 and steam supply 6 to the Calandria 5 were switched off. Sulphuric acid (435 ml, specific gravity 1.84) was added to the water in the storage vessel 1, the stirrer was switched on and milled spent grains were added. During stirring a further 7 litres of water was added to the mixture to give a final volume of 48 litres (acid concentration 1.8% w/w). Steam (0.65 MPa, 160°C) was then admitted to the Calandria 5, the centrifugal pump 2 was switched on and the first valve 3 was opened. A pressure of 0.89 MPa was recorded on the first pressure gauge 4, and the flow of slurry through the apparatus was controlled by adjusting the second valve 11 until the second pressure gauge 10 also registered 0.89 MPa. The residence time of the slurry in the apparatus (i.e. from the pump 2 to the second valve 11) was ca. 15 minutes and the temperature of the reaction mixture measured at 8 varied from 135°C to 140°C throughout the run. The water and reaction product were allowed to run to waste during the first 30 minutes of the run through the third valve 15. This valve was then closed and 15 litres of hydrolyzate were collected and treated with lime and filtered in a similar way to that described in Example 4. The remainder of the slurry was run to waste. A typical composition of broth obtained in this way is shown in Table 7.

TABLE 7

Composition of a Broth obtained by the Hydrolysis of Spent
Grains at Elevated Temperature using a Continuous System
(g/100g Spent Grains on a Dry Weight Basis)

| | |
|---|---|
| Total Carbohydrates | 26 |
| Arabinose | 5 |
| Xylose | 7 |
| Glucose | 13 |
| Total Nitrogen | 0.8 |
| ∝-Amino Nitrogen | 0.1 |
| Colour (EBC Units) | 25 |

EXAMPLE 6

Production of a Broth by Pretreatment of Spent Grains
With Alkali and Digestion with Commercial Cellulase

Samples of wet spent grains (2kg) were boiled under
reflux with 4 litres of sodium hydroxide (3% w/v) for three
hours. After cooling to below 35°C, the pH of the mixture
was adjusted to 5.5 with 2M hydrochloric acid, and the
mixture was diluted to 25 litres with deionized water. The
mixture was then treated with 25,000 units of cellulase CP
from Penicillium funiculosum (John E. Sturge Ltd., Selby,
Yorkshire, U.K.) and maintained at 37°C with continuous
stirring for sixteen to twenty hours. The broth was
separated from the insoluble residue by centrifugation
(6500g) for thirty minutes and the supernatant concentrated
to 2.5 litres by rotary evaporation. The composition of the
resulting brown coloured broth is shown in Table 8.

## TABLE 8

Composition of a Broth obtained by Pretreatment of Spent
Grains with Alkali and Digestion with Commercial
Cellulase (g/100g Spent Grains)

| | |
|---|---|
| Total Carbohydrate | 34.1 |
| Glucose | 13.8 |
| Fructose | 0.6 |
| Sucrose | 0.8 |
| Maltose | 0.1 |
| Total Nitrogen | 2.5 |
| $\alpha$-Amino Nitrogen | 0.2 |
| Colour (E.B.C. Units) | > 100 |

## EXAMPLE 7

### Extraction of Lipids from Residue Obtained after Hydrolysis

The solids remaining after acid hydrolysis of spent grains were dried at 65°C to 5.2% moisture. A portion of these solids (303.3g) was packed into the column of a laboratory scale extractor (see Grimmett, M.Phil. Thesis: Council for National and Academic Awards, 1982, 33) and extracted with liquid carbon dioxide (temperature -5°C, flow 1.35 kg/h) for six hours. The extract (16.2g; 5.6% of the dry weight of solids presented to the extractor) was obtained by opening the tap at the bottom of the evaporator. Analysis of the extract (see Morrison et al., Journal of the Science of Food and Agriculture, 1980 55, 329) showed that 95.6% was lipid and was composed of free fatty acids (50.8%) and triglycerides (36.4%) together with lesser amounts of diglycerides, steryl esters, phospho - and glycolipids and monoglycerides.

## EXAMPLE 8

### Brewing Trials

Pilot-scale brewing trials were carried out using the standard method described by Laws et al, (Journal of the American Society of Brewing Chemists, 1976, 34, 166). In an experimental brew, 10 litres of broth obtained by the acid hydrolysis of spent grains at atmospheric pressure (see Example 1) were added to a kettle containing 40 litres of wort (prepared from 8.2kg of an ale malt) 10 minutes before the end of boiling. This represented a replacement of 10% of the malt by the broth. The specific gravity of the resulting wort was adjusted to 1.040 and subsequent fermentation was satisfactory using yeast NCYC 240. A 3-glass taste test (see Bengtsson, Wallerstein Laboratory Communications, XVl, 54, 231) with 48 tasters was used to compare the flavour of the resulting beer with that of a control beer brewed from 100% of ale malt. Only 19 tasters made the correct selection and hence no significant difference in flavour was detected.

For a *significant difference (5% probability that result due to chance) 22 tasters would have to make the correct solution; for a **significant difference (1% probability of chance result) 25 tasters; and for a ***significant difference (0.1% probability of chance result) 27 tasters would have to be correct.

## EXAMPLE 9

### Ethanol Production from a Broth obtained by the Acid Hydrolysis of Spent Grains at Elevated Temperature

Broth (1.5 litres), produced by the process described in Example 5, and decolorized by filtering through activated charcoal (25g per litre), was fermented under stirred conditions at 20°C in a 2 litre glass fermenter. A flow of oxygen-free nitrogen (40 ml per minute) was maintained through the fermenter head space. The broth was air saturated before inoculation with aerobically grown Saccharomyces cerevisiae NCYC 1342 (2.5g wet weight per litre). Samples for analysis were removed aseptically by a displacement method. Yeast was removed from the samples by centrifugation. The samples were then filtered ( 0.2μ pore size filter) and the monosaccharides present in the filtrate were quantified by high performance liquid chromatography. Ethanol present in the filtrate was determined by gas chromatography (see Jamieson, Journal of the American Society of Brewing Chemists, 1979 37, 151-152). Glucose in the fermentation broth was depleted after 70 hours. 12.6g per litre of ethanol were produced during the fermentation, constituting 82.4% of the maximum theoretical yield. Similar fermentations have been satisfactorily carried out using broth which was not decolorized with activated charcoal.

Ethanol produced by the fermentation of broth from the acid hydrolysis of spent grains could be recovered by standard procedures (distillation etc.).

- 20 -     0169068

CLAIMS:

1.    A method for the utilization of residual carbohydrates in spent grains and/or malting waste products, characterised in that said spent grains and/or malting waste products are hydrolysed at elevated temperature, into a fermentable product.

2.    A method as claimed in Claim 1 characterised in that hydrolysis is carried out under acidic or basic conditions at superatmospheric pressure at a temperature from 120 to 200°C.

3.    A method as claimed in Claim 2 characterised in that hydrolysis is conducted in the presence of 1 to 2 wt. % of sulphuric acid.

4.    A method as claimed in Claim 1 characterised in that hydrolysis is carried out under acidic or basic conditions at atmospheric pressure.

5.    A method as claimed in Claim 4 characterised in that hydrolysis is conducted by boiling with from 1 to 30 wt. % of sulphuric acid for 1 to 6 hours.

6.    A method as claimed in Claim 5 characterised in that the product is filtered to remove insoluble materials.

7.    A method as claimed in any preceding claim characterised in that the fermentable product is subjected to an alcoholic fermentation.

8.    A method as claimed in Claim 7 characterised in that the fermentable product is added to a wort for fermentation.

9. A method as claimed in Claim 7 characterised in that the fermentable product is added to or used as a mashing liquid.

10. A method as claimed in Claim 7 characterised in that the fermentable product is fermented by itself.

0169068

1/2

FIGURE 1

FIGURE 2

0169068